# EUROPÄISCHE PATENTANMELDUNG

(11) **EP 1 878 410 A1**
(43) Veröffentlichungstag der Anmeldung: **16.01.2008**
(21) Anmeldenummer: 07075484.1
(22) Anmeldetag: 18.06.2007
(51) Int. Cl.: A61F 5/41, A61H 1/02

(54) **Streckgerät zur Penisverlängerung**

(30) Priorität: 13.07.2006 DE 202006011087 U
(71) Anmelder: MSP Concept GmbH & Co. KG, 10719 Berlin (DE)
(72) Erfinder: Suchy, Matthias, 10719 Berlin (DE)
(74) Vertreter: Specht, Volker

(57) **Zusammenfassung**

Bei einem Streckgerät zur Penisverlängerung umfasst das Penisbefestigungsmittel (3) eine Aufnahmeschale (14) mit Schlitzöffnungen und ein flexibles, mittels Raststegen (18c) in den Schlitzöffnungen fixierbares Spannelement (18). An der Aufnahmeschale sind den Schlitzöffnungen zugeordnete, schwenkbare Rasthebel (19) zur sicheren Fixierung des Spannelements angebracht.

## Beschreibung

Die Erfindung betrifft ein Streckgerät zur Penisverlängerung durch Gewebewachstum aufgrund einer kontinuierlich auf den Penis wirkenden Streckkraft, das einen Stützring mit gelenkig befestigten, längenverstellbaren Streckstangen und ein am distalen Ende der Streckstangen lösbar gehaltenes Penisbefestigungsmittel, bestehend aus einer Aufnahmeschale mit Schlitzöffnungen und einem flexiblen, mittels Raststegen in den Schlitzöffnungen fixierbaren Spannelement, umfasst.

Eine Vorrichtung zur Penisverlängerung der oben genannten Art wird in der WO 03/073967 A1 beschrieben. Der Penis wird an seinem vorderen Ende in der konkav gewölbten Innenfläche der Aufnahmeschale abgestützt und an dieser mittels des Spannelements fixiert. Das Spannelement hat ein weichelastisches, gegebenenfalls leicht gewölbtes Halteteil und von diesem ausgehende Spannbänder, die jeweils mit mehreren integral angeformten, parallel angeordneten Raststegen ausgebildet sind. Die beiderseitigen Spannbänder mit den Raststegen werden durch die Schlitzöffnungen der Aufnahmeschale gezogen, und zwar bis das jeweilige Spannband in der gewünschten Lage zwischen zwei Raststegen an der Kante der Schlitzöffnungen verrastet. Diese Art der Penisfixierung bei einem Penisverlängerungsgerät ist insofern nachteilig, als das Einfädeln der Spannbänder in die Schlitzöffnungen eine gewisse Geschicklichkeit erfordert und daher mit Schwierigkeiten verbunden ist und die elastischen Spannbänder aufgrund der Kraft- und Reibwirkung an den Raststegen beim Einfädeln bzw. Verrasten oder beim Herausziehen beschädigt werden können. Eine Erhöhung der Festigkeitseigenschaften des Spannelements erhöht auch dessen Steifigkeit, so dass ein schonendes, weichelastisches und möglichst kreisförmiges Umschließen des Penis nicht gewährleistet ist. Zudem kann es beim Festspannen zum Einklemmen von Hautpartien kommen.

Der Erfindung liegt die Aufgabe zugrunde, das Penisbefestigungsmittel für ein Streckgerät der eingangs erwähnten Art so weiterzuentwickeln, dass ein einfaches, komfortables Anlegen des Befestigungsmittels und eine schonende, sichere Fixierung des Penis während des Streckvorgangs gewährleistet ist.

Erfindungsgemäß wird die Aufgabe mit einem gemäß den Merkmalen des Schutzanspruchs 1 ausgebildeten Streckgerät gelöst. Vorteilhafte Weiterbildungen der Erfindung sind Gegenstand der Unteransprüche.

Der Grundgedanke der Erfindung besteht bei einem Penisbefestigungsmittel für ein gattungsgemäßes Streckgerät in der Anordnung jeweils eines an der Außenseite der Aufnahmeschale gelenkig befestigten Rasthebels, dessen Rasthebelarm in der gewünschten Position des Spannelements zwischen zwei Raststegen positioniert und unter Federwirkung gehalten wird bzw. durch manuelle Einwirkung des Benutzers auf einen Betätigungshebelarm des Rasthebels entgegen der Federwirkung das arretierte Spannelement freigibt oder die Schlitzöffnung in der Aufnahmeschale vollständig öffnet. Die Schlitzöffnungen sind mindestens so breit wie der in diese eingeführte Teil des Spannelements zusammen mit den Raststegen dick ist. Aufgrund der erfindungsgemäßen Weiterentwickelung der Aufnahmeschale kann das Befestigungsmittel einfach, bequem und sowohl material- als auch penisschonend angelegt und auch wieder gelöst werden. Für das Spannelement kann ein sehr weichelastisches Material verwendet werden, und zwar ohne dieses beim Ein- oder Ausfädeln zu belasten oder zu beschädigen. Die Spannwirkung des Spannelements kann beim Anlegen des Streckgeräts individuell und bequem eingestellt werden. Die Aufnahmeschale kann so geformt und die Schlitzöffnungen können so beabstandet sein, dass das Penisbefestigungsmittel der Kreisform sehr nahe kommt und dadurch eine sichere, stabile und schonende Halterung des Penis gewährleistet ist.

Der um eine in Lagerböcken gehaltene Achse schwenkbare Rasthebel ist mit einem durch Fingerdruck betätigten Betätigungshebelarm und einem Rasthebelarm ausgebildet. Durch manuelle Einwirkung des Benutzers auf den Betätigungshebelarm wird der Rasthebelarm ausgeschwenkt und dadurch die Arretierung des Spannelements auf einfache Weise gelöst bzw. die Schlitzöffnung zum Einführen des Spannelements vollständig freigegeben. Die Breite des Rasthebelarms entspricht vorzugsweise mindestens der Breite der Raststege, während die Dicke des Rasthebelarms im Bereich der Raststege geringfügig kleiner als der Abstand zwischen diesen ist.

Ein Ausführungsbeispiel der Erfindung wird anhand der Zeichnung näher erläutert. Es zeigen:
- Fig. 1: eine perspektivische Ansicht eines Streckgeräts zur Penisverlängerung;
- Fig. 2: eine Draufsicht auf das aus einer starren Aufnahmeschale und einem - nur teilweise dargestellten - elastischen Spannelement bestehende Penisbefestigungsmittel; und
- Fig. 3: eine Vorderansicht des Penisbefestigungsmittels mit abweichend von der Darstellung in Fig. 1 ausgebildeter Aufnahmeschale.

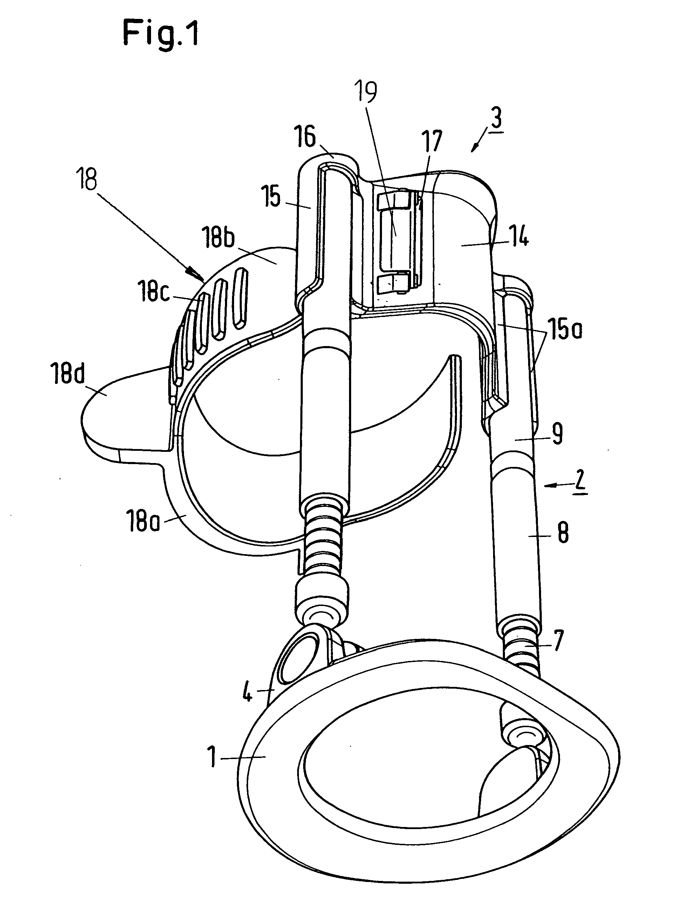

Wie aus Fig. 1 ersichtlich umfasst das Streckgerät einen zur Abstützung am Körper des Benutzers vorgesehenen Stützring 1, zwei an diesem in Gelenken 4 schwenkbar gelagerte, graduell verlängerbare und axial federnd ausgebildete Streckstangen 2 sowie ein am distalen Ende der Streckstangen 2 lösbar gehaltenes Penisbefestigungsmittel 3. Die Streckstangen 2 bestehen jeweils aus einer mit dem Gelenk 4 verbundenen Gewindestange (nicht dargestellt), einer auf dieser axial verstellbaren Verstellhülse 7 mit Federhülse 8 sowie einer Verlängerungsstange 9.

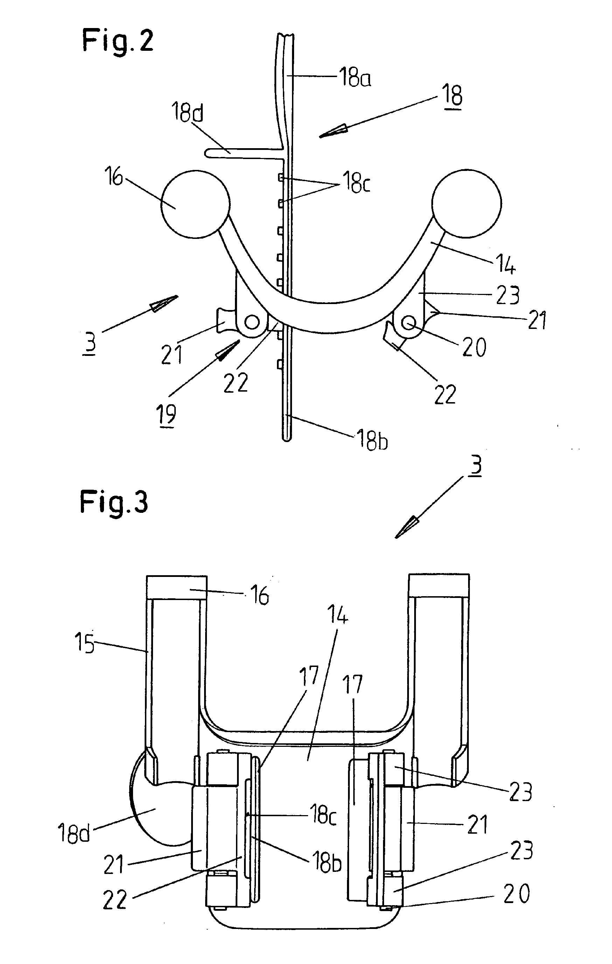

Das Penisbefestigungsmittel 3 besteht zum einen aus einer im Bereich der Auflagefläche des Penis konkav gewölbten, im Wesentlichen biegesteifen Aufnahmeschale 14 mit seitlich angeformten Halteclips 15, die federnde Wangen 15a und ein zylindrisches Steckteil 16 aufweisen. In der Aufnahmeschale 14 sind im Abstand parallel zueinander angeordnete Schlitzöffnungen 17 ausgebildet, denen jeweils ein an der konvex geformten Außenseite der Aufnahmeschale 14 angelenkter, unter Federwirkung stehender Rasthebel 19 zugeordnet ist. Der um eine Achse 20 schwenkbare Rasthebel 19 umfasst einen Betätigungshebelarm 21, mit dem der Rasthebel 19 manuell entgegen einer Federwirkung aus einer Raststellung herausgeschwenkt wird, und einen unter Federkraft in Richtung der Schlitzöffnung 17 wirkenden Rasthebelarm 22. Die Achsen 20 sind zwischen zwei an die Aufnahmeschale 14 angeformten Lagerböcken 23 gehalten.
Das andere Teil des Penisbefestigungsmittel 3 ist ein gummielastisches Spannelement 18, das aus einem weichelastischen Halteteil 18a, zwei Spannbändern 18b mit auf deren Oberfläche im Abstand ausgeformten Raststegen 18c und zwei den Spannbändern 18b entgegen gerichteten Zuglaschen 18d besteht. Die Schlitzöffnungen 17 sind so breit dimensioniert, dass die Spannbänder 18b mit den Raststegen 18c bequem eingezogen werden können. Aus dem gleichen Grund kann auf die Zuglaschen 18d auch verzichtet werden. In den Figuren 2 und 3 ist nur ein Teil des Spannelements 18 dargestellt, dessen Spannband 18b in eine Schlitzöffnung 17 gezogen ist und zwischen zwei Raststegen 18c durch den Rasthebel 19 unter Federwirkung arretiert ist. In der rechten Zeichnungshälfte ist der Rasthebel 19 in der geöffneten, manuell gehaltenen Stellung zum bequemen Einführen des Spannbandes 18b in die freie Schlitzöffnung 17 dargestellt.

### Bezugszeichenliste

- 1: Stützring
- 2: Streckstange
- 3: Penisbefestigungsmittel
- 4: Gelenk
- 7: Verstellhülse
- 8: Federhülse
- 9: Verlängerungsstange
- 14: Aufnahmeschale
- 15: Halteclip
- 15a: federnde Wangen
- 16: Steckteil
- 17: Schlitzöffnung
- 18: elastisches Spannelement
- 18a: weichelast. Halteteil
- 18b: Spannbänder
- 18c: Raststege
- 18d: Zuglaschen
- 19: Rasthebel
- 20: Achse
- 21: Betätigungshebelarm
- 22: Rasthebelarm
- 23: Lagerböcke

## Patentansprüche

1. Streckgerät zur Penisverlängerung durch Gewebewachstum aufgrund einer kontinuierlich auf den Penis wirkenden Streckkraft, das einen Stützring (1) mit gelenkig befestigten, längenverstellbaren Streckstangen (2) und ein am distalen Ende der Streckstangen (2) lösbar gehaltenes Penisbefestigungsmittel (3), bestehend aus einer Aufnahmeschale (14) mit Schlitzöffnungen (17) und einem flexiblen, mittels Raststegen (18c) in den Schlitzöffnungen (17) fixierbaren Spannelement (18), umfasst, **gekennzeichnet durch** an der Außenseite der Aufnahmeschale (14) angelenkte, den Schlitzöffnungen (17) zugeordnete Rasthebel (19) zur Freigabe der Schlitzöffnungen (17) in der manuell ausgeschwenkten Rasthebelstellung und zum Fixieren des in die Schlitzöffnung (17) eingefädelten Spannelements zwischen zwei Raststegen (18c) in der unter Federwirkung zur Aufnahmeschale (14) hin zurückgeschwenkten Position der Rasthebel (19), wobei die Breite der Schlitzöffnungen (17) mindestens der Stärke des in diese eingezogenen Teils des Spannelements (18) samt Raststegen (18c) entspricht.

2. Streckgerät nach Anspruch 1, **dadurch gekennzeichnet, dass** die Rasthebel (19) jeweils an einer in Lagerböcken (23) gehaltenen Achse (20) gelagert sind.

3. Streckgerät nach Anspruch 2, **dadurch gekennzeichnet, dass** die Rasthebel (19) jeweils einen Betätigungshebelarm (21) zum manuellen Freigeben der Schlitzöffnungen (17) beim Einfädeln des Spannelements (18) oder zum Entriegeln des mit einem Rasthebelarm (22) des Rasthebels (19) unter Federwirkung arretierten Spannelements (18) umfassen.

4. Streckgerät nach Anspruch 3, **dadurch gekennzeichnet, dass** die Breite des Rasthebelarms (22) mindestens der Länge der Raststege (18c) entspricht.

5. Streckgerät nach Anspruch 4, **dadurch gekennzeichnet, dass** die Dicke des Rasthebelarms (22) im Bereich der Raststege (18c) kleiner als der Abstand zwischen diesen ist.
